# FASCICULE DE BREVET EUROPEEN

(11) **EP 2 145 004 B1**
(45) Date de publication et mention de la délivrance du brevet: **28.10.2015**
(21) Numéro de dépôt: 08763065.3
(22) Date de dépôt: 06.05.2008
(51) Int. Cl.: C12N 15/53, C12N 9/04, C12N 1/19, C12P 7/18, C12G 1/02

(54) **MOYENS POUR RÉDUIRE L'ACCUMULATION D'ACÉTOÏNE DANS DES MILIEUX DE FERMENTATION ALCOOLIQUE**
MITTEL ZUR REDUZIERUNG DES ACETOIN-AUFBAUS IN MEDIEN FÜR ALKOHOLISCHE GÄRUNG
MEANS FOR REDUCING ACETOIN BUILDUP IN ALCOHOLIC FERMENTATION MEDIA

(30) Priorité: 07.05.2007 FR 0703279
(43) Date de publication de la demande: 20.01.2010
(73) Titulaire: Danstar Ferment AG, 6300 Zug (CH); Institut National de la Recherche Agronomique (INRA), 75338 Paris Cedex 07 (FR)
(72) Inventeur: DEQUIN, Sylvie, F-34000 Montpellier (FR); EHSANI, Maryam, F-34000 Montpellier (FR); FÉRNANDEZ GALLEGOS, Maria Rosario, E-08210 Barberà Del Vallès (barcelona) (ES); BIOSCA, Josep A., E-08005 Barcelona (ES); ORTIZ-JULIEN, Anne, F-31150 Gagnac-sur-garonne (FR)
(74) Mandataire: Cabinet Armengaud Aîné
(86) Numéro de dépôt international: PCT/IB2008/051761
(87) Numéro de publication internationale: WO 2008/135950

(56) Documents cités:
- WO-A-96/41888
- GONZALEZ E ET AL: "Characterization of a (2R,3R)-2,3-butanediol dehydrogenase as the Saccharomyces cerevisiae YAL060W gene product. Disruption and induction of the gene" JOURNAL OF BIOLOGICAL CHEMISTRY, AMERICAN SOCIETY OF BIOLOCHEMICAL BIOLOGISTS, BIRMINGHAM,, US, vol. 275, no. 46, novembre 2000 (2000-11), pages 35876-35885, XP002264210 ISSN: 0021-9258 cité dans la demande
- REMIZE F ET AL: "Glycerol overproduction by engineered Saccharomyces cerevisiae wine yeast strains leads to substantial changes in by-product formation and to a stimulation of fermentation rate in stationary phase" APPLIED AND ENVIRONMENTAL MICROBIOLOGY, vol. 65, no. 1, janvier 1999 (1999-01), pages 143-149, XP002462342 ISSN: 0099-2240 cité dans la demande
- GONZALEZ EVA ET AL: "Characterization and functional role of Saccharomyces cerevisiae 2,3-butanediol dehydrogenase", CHEMICO-BIOLOGICAL INTERACTIONS, vol. 130-132, no. 1-3, 30 January 2001 (2001-01-30), pages 425-434, ISSN: 0009-2797
- CAMBON B ET AL: "Effects of GPD1 overexpression in Saccharomyces cerevisiae commercial wine yeast strains lacking ALD6 genes", APPLIED AND ENVIRONMENTAL MICROBIOLOGY, AMERICAN SOCIETY FOR MICROBIOLOGY, US, vol. 72, no. 7, 1 July 2006 (2006-07-01), pages 4688-4694, XP002564241, ISSN: 0099-2240, DOI: 10.1128/AEM.02975-05
- EHSANI MARYAM ET AL: "Engineering of 2,3-Butanediol Dehydrogenase To Reduce Acetoin Formation by Glycerol-Overproducing, Low-Alcohol Saccharomyces cerevisiae", APPLIED AND ENVIRONMENTAL MICROBIOLOGY, AMERICAN SOCIETY FOR MICROBIOLOGY, US, vol. 75, no. 10, 1 May 2009 (2009-05-01), pages 3196-3205, XP002564242, ISSN: 0099-2240, DOI: 10.1128/AEM.02157-08
- REMIZE F ET AL: "GLYCEROL EXPORT AND GLYCEROL-3-PHOSPHATE DEHYDROGENASE, BUT NOT GLYCEROL PHOSPHATASE, ARE RATE LIMITING FOR GLYCEROL PRODUCTION IN SACCHAROMYCES CEREVISIAE", METABOLIC ENGINEERING, ACADEMIC PRESS, US, vol. 3, no. 4, 1 October 2001 (2001-10-01) , pages 301-312, XP008073090, ISSN: 1096-7176, DOI: 10.1006/MBEN.2001.0197

### SEQUENCE LISTING

<110> LALLEMAND SAS INSTITUT NATIONAL DE LA RECHERCHE AGRONOMIQUE (INRA)
<120> MOYENS POUR RÉDUIRE L'ACCUMULATION D'ACÉTOÏNE DANS DES MILIEUX DE FERMENTATION ALCOOLIQUE
<130> CP/SF 62209-3069
<150> FR 07/03279
   <151> 2007-05-07
<160> 9
<170> PatentIn version 3.3
<210> 1
   <211> 60
   <212> DNA
   <213> Artificial sequence
<220>
   <223> oligonucléotide
<400> 1
   ctttcctcct tacggggtcc tagcctgttt ctcttgatat gcaggtcgac aacccttaat 60
<210> 2
   <211> 61
   <212> DNA
   <213> Artificial sequence
<220>
   <223> oligonucléotide
<400> 2
   agtgaatatc acccttcttg aaatatgcca aagctctcat tcgaaactaa gttcttggtg 60
   t 61
<210> 3
   <211> 51
   <212> DNA
   <213> Artificial sequence
<220>
   <223> oligonucléotide
<400> 3
   gggctagtaa aattgtagtg tcttcaattg cagagagaag aatagaaatg g 51
<210> 4
   <211> 52
   <212> DNA
   <213> Artificial sequence
<220>
   <223> oligonucléotide
<400> 4
   ccatttctat tcttctctct gcaattgaag acactacaat tttactagcc cc 52
<210> 5
   <211> 52
   <212> DNA
   <213> Artificial sequence
<220>
   <223> oligonucléotide
<400> 5
   ggggctagta aaattgtagt gtcttcaaga gcagagagaa gaatagaaat gg 52
<210> 6
   <211> 52
   <212> DNA
   <213> Artificial sequence
<220>
   <223> oligonucléotide
<400> 6
   ccatttctat tcttctctct gctcttgaag acactacaat tttactagcc cc 52
<210> 7
   <211> 52
   <212> DNA
   <213> Artificial sequence
<220>
   <223> oligonucléotide
<400> 7
   ggggctagta aaattgtagt gtcttcaaga tcagagagaa gaatagaaat gg 52
<210> 8
   <211> 52
   <212> DNA
   <213> Artificial sequence
<220>
   <223> oligonucléotide
<400> 8
   ccatttctat tcttctctct gatcttgaag acactacaat tttactagcc cc 52
<210> 9
   <211> 55
   <212> DNA
   <213> Artificial sequence
<220>
   <223> oligonucléotide
<400> 9
   ggccatttct attcttctct ctgatcttga agacactaca attttactgg ccccc 55

## Description

L'invention a pour objet des levures et des procédés de fermentation utilisant ces levures, pour réduire l'accumulation d'acétoïne dans des milieux de fermentation alcoolique.

Depuis une quinzaine d'années, les connaissances scientifiques et le savoir-faire en viticulture et en zoologie ont conduit à une amélioration très significative des qualités organoleptiques des vins. Les pratiques viticoles actuelles favorisent la production de vins à potentiel qualitatif élevé en retardant le moment de la vendange. Il en résulte, comme conséquence majeure, une augmentation de la teneur en sucre des moûts, et donc de la teneur en alcool des vins (fréquemment supérieure à 14°). Cette dérive, rencontrée dans la plupart des zones de production, pose ouvertement de nombreux problèmes à la filière viticole mondiale. Des teneurs en alcool excessives sont en effet difficilement compatibles avec les préoccupations de santé et de bien-être des consommateurs, et font par ailleurs l'objet de taxes dans certains pays.

Il existe de ce fait une demande croissante pour des méthodes et outils permettant de réduire la teneur en alcool des vins et d'autres boissons alcoolisées. Les approches physiques (par exemple distillation sous vide) sont de plus en plus utilisées, mais sont difficilement compatibles avec le maintien d'une qualité organoleptique satisfaisante.

Une solution biologique reposerait sur l'utilisation de souches de levures à faible rendement en alcool.

Par exemple, les levures *Saccharomyces cérevisiae,* notamment les levures *Saccharomyces cerevisiae* oenologiques, transforment les sucres en alcool avec un rendement de 0,47 g/g, qui varie peu suivant la souche utilisée. De ce fait, l'obtention d'une levure *Saccharomyces cerevisiae* à faible rendement en alcool nécessite la mise en oeuvre de stratégies génétiques visant à dévier une partie des sucres vers la formation d'autres sous-produits.

Plusieurs approches d'ingénierie génétique ont été mises en oeuvre pour dévier une partie des sucres vers la production d'autres sous-produits que l'éthanol. Ces approches ont été basées sur la modification de l'activité d'enzymes impliquées dans la synthèse de glycérol ou dans l'utilisation du pyruvate. Par exemple, la surproduction de glycérol, obtenue par surexpression de *GPD1* ou *GPD2* codant pour la glycérol 3-phosphate déshydrogénase (Michnick et al, 1997 ; Remize et al, 1999 ; demande de brevet internationale WO 96/41888) a ainsi permis de diminuer la teneur en éthanol de 1 à 2°. Elle s'accompagne toutefois de modifications majeures du niveau de production d'autres métabolites, dont certains indésirables dans le vin, notamment l'acétate et l'acétoïne (3-hydroxy-2-butanone). La production d'acétate peut être réduite par délétion de *ALD6* codant pour une acétaldéhyde déshydrogénase. Par contre, aucune stratégie n'a été pour l'instant décrite permettant de réduire l'accumulation d'acétoïne. Ce composé est accumulé à raison de plusieurs grammes par litre, alors qu'il est défavorable, par exemple dans le vin, à plus de 150 mg/L (seuil de détection olfactif).

Remize F. et al., ("Glycerol overproduction by engineered Saccharomyces cerevisiae wine yeast strains leads to substantial changes in by-formation and to a stimulation of fermentation rate in stationary phase », Applied and Environmental Microbiology, vol 65, No. 1, janvier 1999, pages 143-149) décrit une souche de levure *Saccharomyces cerevisiae* génétiquement modifiée, dans laquelle le gène GPD1, codant pour une glycérol-3-phosphate déshydrogénase, est surexprimé. Cette souche produit du glycérol et du 2,3-butanediol à partir de l'acétoïne.

Cambon B. et al., (« Effects of GPD1 overexpression in Saccharomyces cerevisiae commercial wine yeast strains lacking ALD6 genes », Applied and Environmental Microbiology, vol 72, No. 7, 1 juillet 2006, pages 4688-4694) décrit l'utilisation d'une souche de levure *Saccharomyces cerevisiae* surproductrice de glycérol pour la production de vin ayant une teneur diminuée en éthanol. Les auteurs ont évalué les effets de la surexpression du gène GPD1 codant pour une glycérol-3-phosphate déshydrogénase dans des souches de levures dans lesquelles deux copies du gène ALD6 ont été supprimées. Le produit obtenu par ces souches de levures modifiées contient une forte teneur en acétoïne du fait de l'incapacité des souches à réduire l'acétoïne en 2,3-butanediol.

La solution proposée par les inventeurs consiste à convertir l'acétoïne produit en 2,3-butanediol, composé considéré comme neutre d'un point de vue organoleptique, son seuil de détection dans le vin étant supérieur à 12 g/L.

La production de 2,3-butanediol à partir de l'acetoïne est réalisée par l'enzyme butanediol déshydrogénase (Bdh1p) codée par *BDH1.*

La voie de production de 2,3-butanediol contribue à l'équilibre rédox NAD⁺/NADH intracellulaire. Chez les bactéries, cette voie peut également intervenir dans la régulation du pH intracellulaire.

Le 2,3-butanediol est produit sous plusieurs formes : (2R,3R)-2,3-butanediol et (2S,3S)-2,3-butanediol, deux formes optiquement actives, (2R,3S)-2,3-butanediol et (2S,3R)-2,3-butanediol, correspondant aux formes *méso.*

La butanediol déshydrogénase codée par *BDH1* est la principale enzyme impliquée dans la production de 2,3-butanediol. Elle est responsable de la formation de la totalité du (2R,3R)-2,3-butanediol et d'une partie du (méso)-2,3-butanediol, à partir de R-acétoïne et S-acétoïne, respectivement. Bdh1p ayant d'autre part une plus forte affinité pour l'acétoïne (Km_{acétoïne} : 4,5 mM, Km_{2,3-butanediol} : 14 mM), la réaction est fortement déplacée dans le sens de la formation de 2,3-butanediol.

Les publications de Gonzalez E. et al., («Characterization of a (2R,3R)-2,3-Butanediol dehydrogenase as the Saccharomyces cerevisiae YAL060W gene product », The Journal of Biological Chemistry, vol 275, No.46, 17 Novembre 2000, pages 35876-35885 et «Characterization and functional role of Saccharomyces cerevisiae 2,3-butanediol dehydrogenase », Chemico-Biological Interactions, vol 130-132, No.1-3, 30 Janvier 2001, pages 425-434) décrivent une souche de levure *Saccharomyces cerevisiae* comportant un gène BDH, codant pour une protéine Bdh, ayant une affinité accrue pour le cofacteur NADH, et dans laquelle le gène BDH est surexprimé. Dans les deux publications de Gonzalez, la souche de levure produit du 2,3-butanediol à partir de l'acétoïne. Néanmoins, les publications de Gonzalez *et al.* ne décrivent pas de souches de levures comprenant une ou plusieurs mutations dans le gène BDH1 afin de présenter une spécificité de cofacteur pour le NADPH au lieu du NADH.

Dans l'approche suivie, les inventeurs ont considéré que le niveau de synthèse de la 2,3-butanediol déshydrogénase est un facteur limitant la réaction de conversion de l'acétoïne en 2,3-butanediol. Ils ont également retenu le fait qu'une moindre disponibilité en NADH pourrait empêcher une conversion plus efficace de l'acétoïne. Une stratégie de surexpression de *BDH1* et de mutagénèse dirigée de *BDH1* a été alors mise en oeuvre pour changer sa spécificité de cofacteur de NADH à NADPH.

Leurs travaux ont ainsi permis de transformer des souches de *Saccharomyces* et de les rendre capables de convertir efficacement de l'acétoïne en 2,3-butanediol au cours d'une fermentation alcoolique, même lorsque l'acétoïne est produit en quantités importantes (plusieurs g/l).

L'invention a donc pour but de fournir de nouvelles souches de levures génétiquement transformées pour surexprimer *BDH1* et présentant une spécificité modifiée de cofacteur, afin de produire de plus grandes quantités de 2,3-butanediol à partir d'acétoïne.

L'invention vise également à fournir un procédé d'obtention de telles souches.

Elle a également pour but la mise à profit des propriétés de ces souches de levures transformées dans un procédé de fermentation alcoolique ainsi que pour la production de 2,3-butanediol.

Les souches de levure de l'invention sont des levures surexprimant, par rapport à la souche initiale, le gène *BDH1* codant pour Bdh1p.

Ces souches sont caractérisées en ce qu'elles catalysent dans un milieu de fermentation alcoolique la réduction de l'acétoïne en 2,3-butanediol selon un taux au moins 2 fois supérieur à celui de la souche initiale.

Il s'agit plus spécialement de souches génétiquement transformées et/ou mutées de manière à obtenir un taux de conversion d'acétoïne en 2,3-butanediol d'au moins 2 fois supérieur à celui de la souche initiale.

Par « souche initiale», on entend une souche avant surexpression ou modification du gène *BDH1.*

L'invention vise ainsi des souches de levures telles que définies ci-dessus, génétiquement transformées pour surexprimer *BDH1,* à l'aide de séquences régulatrices propres à augmenter l'expression dudit gène. De telles souches transformées contiennent au moins 2 copies du gène *BDH1.* On peut utiliser tout promoteur actif chez l'hôte dans lequel on souhaite obtenir l'expression de ce gène, de préférence des promoteurs décrits comme forts (codant pour des gènes fortement exprimés) en conditions de fermentation alcoolique. C'est le cas par exemple de gènes glycolytiques fortement exprimés en fermentation comme *ADH1* (*alcool* déshydrogénase), *PGK1* (phosphoglycérate kinase), *TDH3* (glycéraldéhyde déshydrogénase), ou du facteur de transcription *TEF1,* mais il en existe de nombreux autres.

L'invention vise également des souches dans lesquelles le gène *BDH1* comporte une ou plusieurs mutations de manière à coder pour une protéine Bdh1p dans laquelle un ou plusieurs acides aminés sont mutés.

De manière avantageuse, de telles souches comprennent une ou plusieurs mutations dans le gène *BDH1* afin de présenter une affinité accrue pour le çofacteur NADPH au lieu du NADH.

Le choix des acides aminés à modifier repose sur le fait que le NAD(H) diffère du NADP(H) dans le groupement phosphate estérifié à la position 2' du ribose d'adénosine. De ce fait, les acides aminés interagissant avec ce groupement sont des candidats pour le changement de spécificité de cofacteur. Le résidu déterminant de la spécificité de NAD(H) est l'aspartate (Asp) ou le glutamate (Glu), qui forment des liaisons hydrogène avec les groupements hydroxyle 2' et 3' dans la partie ribosyle du coenzyme. Les déshydrogénases NADP(H)-dépendantes ont un résidu plus petit et neutre tel que la glycine (Gly), l'alanine (Ala) et la serine (Ser), à la même position. De plus, un résidu d'arginine (Arg) adjacent permet une bonne interaction avec le groupement phosphate de NADP(H).

Ainsi, des remplacements avantageux conformément à l'invention concernent le résidu Glu(E)221 par Ser(S), le résidu Ile (I) 222 par Arg (R) et le résidu Ala(A)223 par Ser, en se basant sur la structure de l'enzyme NADPH-dépendante Adh6p de *S. cerevisiae* (les positions sont notées selon la séquence en acides aminés de Bdh1p de *Saccharomyces cerevisiae S288C*).

La (les) modification(s) de Glu (Asp) 221 et/ou Ile (val) 222, et/ou Ala223 et toute combinaison possible de ces 3 modifications entrent également dans le champ de l'invention.

Le premier résidu peut être également une sérine (comme dans l'exemple), ou tout acide aminé petit et neutre comme Gly ou Ala.

L'invention vise aussi des souches telles que définies ci-dessus génétiquement modifiées pour surexprimer *BDH1* dans un milieu de fermentation alcoolique à l'aide d'un promoteur fort de levures et comprenant une ou plusieurs mutations dans *BDH1* afin de présenter une spécificité de cofacteur pour le NADPH au lieu du NADH.

Dans un mode préféré de réalisation de l'invention, les souches de levure qui produisent de l'acétoïne sont des souches surproductrices de glycérol. Il s'agit notamment de souches contenant le gène *GPD1* ou le gène *GPD2* surexprimé, par exemple obtenu par transformation des souches par le plasmide PVT100U-*ZEO-URA3-GPD1* ou par changement *in situ* de son promoteur par un promoteur fort.

Dans un autre mode de réalisation de l'invention, les souches de levure sont génétiquement modifiées de manière à réduire la production d'acétate (en plus de la surexpression de *GPD1*). Plus particulièrement, il s'agit de souches dans lesquelles le gène *ALD6* et le cas échéant ses copies, a (ont) été délété(es). été délété(es).

Des souches préférées appartiennent au genre *Saccharomyces* et comprennent, notamment, les espèces *Saccharomyces cerevisiae, Saccharomyces bayanus, Saccharomyces uvarum,* et *Saccharomyces kudriavzevïi.*

D'autres souches préférées appartiennent au genre non *Saccharomyces.*

L'invention vise également les hybrides des souches définies ci-dessus.

L'invention vise également un procédé d'obtention des souches de levures ci-dessus mentionnées.

Ce procédé met avantageusement à profit les techniques du génie génétique et de mutagénèse dirigée. On mentionnera à titre d'exemple l'utilisation d'un oligonucléotide comportant les mutations cibles souhaitées pour modifier le gène *BDH1* d'une souche de levure, et la transformation de la souche avec un fragment amplifié, l'amplification d'une région du gène comportant les mutations, ou encore le croisement entre souches, en partant d'une souche comportant par exemple les mutations désirées pour les transférer dans une autre.

L'invention a également pour objet un procédé de fermentation, caractérisé par l'addition d'une souche de levure transformée telle que définie ci-dessus au milieu de fermentation.

Le milieu de fermentation est notamment un moût, avantageusement un jus de raisin.

Selon un aspect de grand intérêt, le 2,3-butanediol obtenu en utilisant les souches de levure de l'invention constitue un composé important pour une variété de matières de base chimiques et carburants liquides. Ce composé peut conduire par déshydratation la formation de méthyléthyl cétone, utilisable en tant qu'additif de carburant liquide.

Le 2,3-butanediol peut également être converti en 1,3-butanediène, composé utilisé dans la production de gomme synthétique. D'autres dérivés, pour des utilisations, par exemple, comme agents antigel (forme lévo), solvants et plastiques, peuvent aussi être préparés à partir de 2,3-butanediol. En plus, il peut être ajouté à des produits alimentaires en tant qu'arôme après conversion en diacétyle par déshydrogénation.

L'estérification de butanediol amène vers la formation de précurseurs de polyuréthane pour utilisation dans des médicaments, produits cosmétiques et lotions etc.

L'utilisation des souches de levure pour produire du 2,3-butanediol notamment dans les applications mentionnées ci-dessus entre aussi dans le champ de l'invention.

En particulier, l'invention fournit des moyens pour obtenir un rendement et une productivité plus importants en 2,3-butanediol de manière générale, mais également pour obtenir des stéréoisomères purs, en quantités importantes au lieu d'un mélange d'isomères.

Les souches de levure de l'invention permettent également de réduire le taux de diacétyle produit. Elles sont avantageusement utilisées à cet effet, notamment dans les boissons fermentées.

D'autres caractéristiques et avantages de l'invention sont donnés dans les exemples qui suivent de modes de réalisation. Dans ces exemples, il est fait référence aux figures 1 à 7 qui représentent, respectivement :
- **Fig. 1** **:** l'effet de la surexpression de *BDH1* sur la production d'acétoine par la souche V5*ald6 BDH1 pGPD1* pour des concentrations en glycérol variables ;
- **Fig. 2** **:** la production d'acétoïne (a et a') et de 2,3-butanediol (b et b') au cours de la fermentation pour les souches V5 et V5*ald6 pGPD1* et V5*ald6 BDH1 pGPD1* dans conditions (i) : a, b et (ii) : a', b' ;
- **Fig. 3** **:** l'alignement de séquences de *BDH1* (en gras) et *BDH1*₂₂₃. En gris : le changement des 3 résidus acides aminés E221S/V222R/A223S induisant une réversion de la spécificité du cofacteur chez Bdh1p ;
- **Fig. 4** **:** la formation d'acétoïne dans les souches V5*ald6 BDH1 pGPD1* (noir) et V5*ald6 BDH1*₂₂₃ *pGPD1* (blanc) au cours d'une fermentation ;
- **Fig. 5** **:** la formation finale d'acétoïne dans les souches CEN.PK*ald6 pGPD1* (blanc), CEN.PK*ald6 EDH1 pGPD1* (gris) et CEN.PK*ald6 BDH1*₂₂₃ *pGPD1* (noir) à l'issue de plusieurs fermentations ;
- **Fig. 6** **:** le fonctionnement de formes mutantes *in vivo ;* et
- **Fig. 7** **:** la réduction de la formation de diacétyle par surexpression de *BDH1.*

### • Obtention de souches Saccharomyces cerevisiae surexprimant BDH1

Le promoteur de *BDH1* a été remplacé par le promoteur de *TDH3 in situ,* en utilisant la technique de Short Flanking Homologous (SFH) PCR (Polymerase Chain Reaction) (Guldener et al., 1996).

Un fragment constitué du module kanMX, qui porte le gène kanR conférant la résistance à l'antibiotique G418^{R}, et du promoteur *TDH3* a été amplifié par PCR à partir du plasmide bactérien pUG6-NOXE (Heux *et al.,* 2005) à l'aide des oligonucléotides de séquences, respectivement, SEQ ID N°1 et SEQ ID N°2, portant une séquence homologue à pUG6-NOXE et une séquence flanquante (en italique) homologue à la région cible chromosomique (promoteur *BDH1*).
SEQ ID N° 1 : 5' *CTTTCCTCCT TACGGGGTCC TAGCCTGTTT CTCTTGATAT* GCAGGTCGAC AACCCTTAAT 3'
SEQ ID N°2 : 5' *AGTGAATATC ACCCTTCTTG AAATATGCCA AAGCTCTCAT* TCGAAACTAA GTTCTTGGTGT 3'
Conditions expérimentales utilisées pour la PCR:

| | |
|---|---|
| Oligonucléotide SEQ ID N°1 | 5µl (20 pmoles) |
| Oligonucléotide SEQ ID N°2 | 5µl (20 pmoles) |
| DyNazyme EXT Buffer 10X + 15 mM MgCl₂ (FINNZYMES, Finlande) | 5µl |
| dNTP 2 mM | 5µl |
| pUG6-NOXE | 1µl (25 ng) |
| H₂O | 28,25µl |
| DyNazyme EXT (FINNZYMES, Finlande) | 0,75µl |

30 cycles (45 sec 94°C, 30 sec 55°C, 2 min 72°C) sur un amplificateur Perkin-Elmer Cetus modèle 9600.

Le produit de PCR obtenu a été précipité à l'éthanol en présence de sels. 3µg de l'ADN précipité ont été utilisés pour transformer les souches de levure *S. cerevisiae* V5 et *V5ald6.* La souche V5 (*MAT**a**, ura3*) est dérivée d'une souche oenologique. La souche V5 et la souche V5*ald6* (Remize *et al.,* 1999) dans laquelle les deux copies du gène *ALD6* ont été délétées ont été transformées par la méthode d'acétate de lithium (Schiestl et Gietz, 1989). Les transformants ont été sélectionnés sur boîtes YPD G418^{R}. L'intégration du promoteur *TDH3* à la place du promoteur *BDH1* a été vérifiée par PCR.

### Résultats

Les résultats obtenus montrent que la souche V5 *BDH1* a la même croissance et vitesse de fermentation que la souche témoin V5. La mesure de l'activité BDH au cours de la phase exponentielle et de la phase stationnaire montre que l'activité enzymatique BDH de la souche surexprimant *BDH1* est supérieure d'environ 30 fois à celle de la souche sauvage (activité spécifique moyenne V5 : 0,1 U/mg protéine totale ; activité spécifique moyenne V₅ *BDH1 :* 3,2 U/mg protéine totale).

### • Effet de la surexpression de BDH1 dans des souches surexprimant GPD1

Les souches V5, V5*ald6*, V5 *BDH1* et V5*ald6 BDH1* ont été transformées par 10 ng du vecteur multicopie PVT100U-ZEO-*GPD1* (Remize *et al,* 1999) portant le gène URA3. Les transformants ont été sélectionnés sur boîtes YNB supplémentées en méthionine (115 mg/L). Après vérification par PCR, quatre souches : V5 *pGPD1,* V5*ald6* p*GPD1,* V5 *BDH1* p*GPD1* et V5*ald6 BDH1 pGPD1* ont été obtenues.

### • Effet de la surproduction de Bdh1p sur la synthèse d'acétoïne

*Conditions de culture -* Les souches étudiées pendant la fermentation en conditions oenologiques sont indiquées dans le tableau 1 ci-après.

**Tableau 1.**

| **Souche** | **Caractéristiques** |
|---|---|
| V5 | Souche *S. cerevisiae* dérivée d'une levure oenologique *MAT***a***, ura3* |
| V5 *BDH1* | Souche V5 surexprimant *BDH1* |
| V5 *pGPD1* | Souche V5 surexprimant *GPD1* |
| V5 *BDH1 pGPD1* | Souche V5 surexprimant *BDH1* et *GPD1* |
| *V5ald6 pGPD1* | Souche V5 délétée pour *ALD6* et surexprimant *GPD1* |
| V5*ald6 BDH1 pGPD1* | Souche V5 délétée pour *ALD6,* surexprimant *GPD1* et *BDH1* |

Les fermentations ont été réalisées dans des réacteurs de 1,2 litres (SGI, France) avec un volume réactionnel de 1 litre. Le milieu MS a été utilisé pour la préculture et la culture. Il s'agit d'un milieu synthétique qui simule un moût standard de raisin (Bely et *al.,* 1990). Le milieu MS contient 20% glucose, 6 g/l d'acide malique, 6 g/l d'acide citrique, 460 mg/l d'azote, sous forme de NH₄Cl (120 mg/l) et d'acides aminés (340 mg/l). Le milieu est supplémenté avec de la méthionine (115 mg/l) et si nécessaire de l'uracile (50 mg/l). Le pH du milieu MS est de 3,3. Des facteurs d'anaérobiose, ergostérol (7,5 mg/l), acide oléique (2,5 mg/l) et Tween 80 (0,21 g/l) sont ajoutés. Les précultures ont été réalisées dans des erlenmeyers de 100 ml contenant 20 ml de milieu à 28°C sous agitation (150 rpm) pendant 30h. Les réacteurs ont été inoculés à partir de ces précultures, à une densité cellulaire de 1.10⁶ cellules/ml, et maintenus à température constante de 28°C avec agitation permanente (300 rpm).

Les échantillons de culture ont été collectés à l'aide d'une seringue. Les données de fermentation sont exprimées en fonction du temps.

*Méthodes analytiques -* La croissance a été suivie par la mesure de la densité optique à 600 nm et par le comptage du nombre de cellules sur un appareil de type Coulter Counter (ZBI) à partir d'une fraction aliquote de milieu de culture.

Les métabolites ont été dosés dans le surnageant, après centrifugation à 13000 rpm pendant 5 minutes des prélèvements réalisés. Les concentrations en glucose, glycérol, éthanol, pyruvate, succinate, acétate, α-cétoglutarate et 2-hydroxyglutarate ont été déterminées par chromatographie liquide sous haute pression (HPLC) en utilisant une colonne de type HPX-87H (Bio-Rad). La concentration en acétaldéhyde a été déterminée par la méthode enzymatique décrite par Lundquist, (1974). Les concentrations en acétoïne et en 2,3-butanediol ont été déterminées par chromatographie en phase gazeuse comme décrit par Michnick *et al*., 1997.

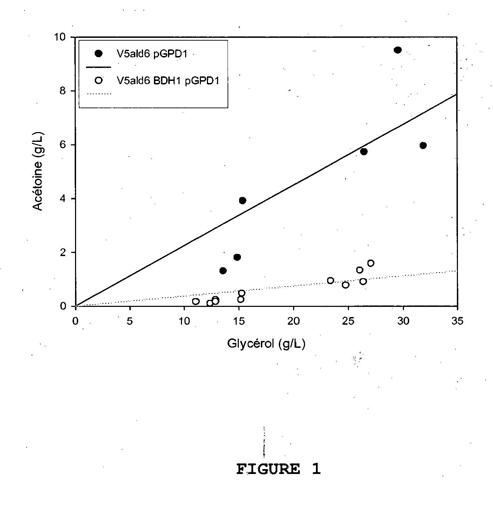

La figure 1 montre l'impact de la surexpression de *BDH1* sur la production d'acétoïne. Dans cet exemple, la production de ce composé a été analysée au cours de la fermentation par V5*ald6 pGPD1* et *V5ald6 BDH1 pGPD1.* Deux conditions différentes de surproduction de glycérol ont été utilisées. En conditions (i) aucune pression de sélection n'a été utilisée pour maintenir le plasmide pVT100U-ZEO-*GPD1,* résultant en une surproduction modérée de glycérol, atteignant 10-15 g/L. En conditions (ii), les souches V5 *BDH1 pGPD1* et *V5ald6 BDH1 pGPD1* ont été cultivées en l'absence d'uracile, permettant le maintien du plasmide tout au long de la fermentation, générant une surproduction très élevée de glycérol, 20-30 g/L.

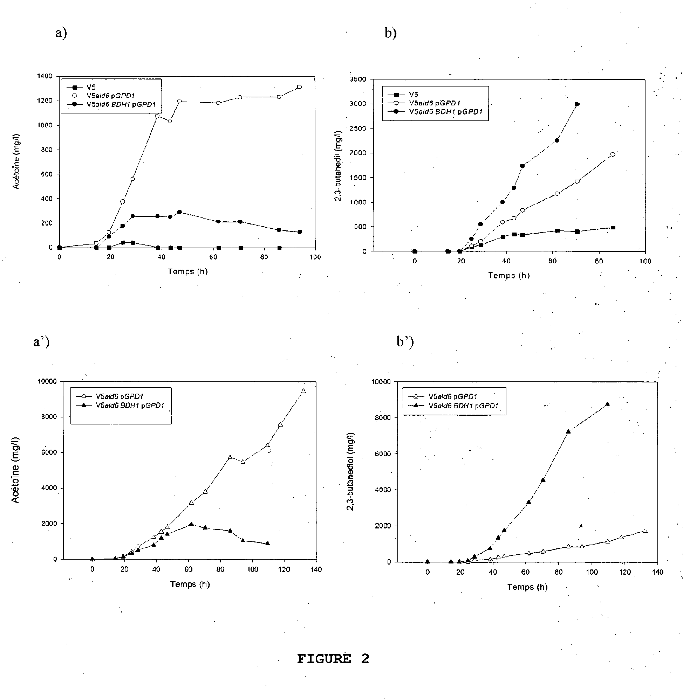

Dans les deux conditions testées (i) et (ii) la surexpression de *BDH1* permet une diminution drastique de la production d'acétoïne jusqu'à 90 % par rapport au niveau produit par la souche témoin *V5ald6 pGPD1,* et une augmentation correspondante de la production de 2,3-butanediol (Fig. 2). La souche V5 a été utilisée comme contrôle. Cette souche n'accumule pas d'acétoïne et produit environ 0,5 g/L de 2,3-butanediol.

La figure 2 montre les niveaux d'acétoïne obtenus après plusieurs expériences de fermentation avec les souches *V5ald6 pGPD1* et *V5ald6 BDH1 pGPD1,* dans les mêmes conditions que ci dessus.

### Mutagénèse dirigée in vitro de BDH1

En partant de la séquence protéique codée par le gène *BDH1* de la souche S288C [Saccharomyces genome database http://www.yeastgenome.org/], des mutations correspondant au changement d'un acide aminé ont été introduites à chaque étape de PCR. Grâce aux oligonucléotides comprenant les mutations souhaitées et un plasmide contenant le *BDH1* natif de S288C (pYES2*-BDH1*), les enzymes mutées suivantes ont été construites :

| | | |
|---|---|---|
| **Enzyme sauvage :** | ***BDH1* de S288C** | **221 EIAERR 226** |
| **Simple mutant :** | **E221S** | **221 SIAERR 226** |
| **Double mutant :** | **E221S/I222R** | **221 SRAERR 226** |
| **Triple mutant :** | **E221S/I222R/A223S** | **221 SRSERR 226** |

### - Mutagénèse dirigée

Les mutations ont été introduites en utilisant une méthode basée sur le Quickchange II XL Site-Directed Mutagenesis Kit (Stratagene, USA). Le mutant E221S a été construit à partir de la région codante de *BDH1* de la souche de laboratoire S288C clonée dans le plasmide pYES2 (Invitrogen, Carlsbad, CA) : pYES2*-BDH1* (Gonzalez E, 2000). Ce vecteur navette contient un promoteur inductible (promoteur et séquence UAS de *GAL1*), l'origine de réplication 2µ, le marqueur de sélection *URA3* et les éléments bactériens (origine de réplication et gène de résistance à l'ampicilline).

Le plasmide pYES2*-BDH1* contenant les mutations E221S, E221S/I222R et E221S/I222R/A223S a été utilisé comme matrice pour obtenir le simple, le double et triple mutant, respectivement.

La mutagénèse a été effectuée par PCR en utilisant des oligonucléotides contenant les mutations (en gras) et dans le sens direct et dans le sens inverse (E221S : MUT 221-1 (SEQ ID N°3), MUT 221-2 (SEQ ID N°4) ; E221S/I222R : MUT 222-1 (SEQ ID N°5), MUT 222-2 (SEQ ID N°6) ; E221S/I222/R/A223S : MUT 223-1 (SEQ ID N°7), MUT 223-2 (SEQ ID N°8)).
SEQ ID N°3 (MUT223221-1)
   5'GGGCTAGTAAAATTGTAGTGTCT**TCA**ATTGCAGAGAGAAGAATAGAAATGG 3',
SEQ ID N°4 (MUT223221-2)
   5'CCATTTCTATTCTTCTCTCTGCAAT**TGA**AGACACTACAATTTTACTAGCCCC 3'
SEQ ID N°5 (MUT224222-1)
   5' GGGGCTAGTAAAATTGTAGTGTCT**TCAAGA**GCAGAGAGAAGAATAGAAATGG 3'
SEQ ID N°6 (MUT224222-2)
   5' CCATTTCTATTCTTCTCTCTGC**TCTTGA**AGACACTACAATTTTACTAGCCCC 3'
SEQ ID N°7 (MUT225223-1)
   5' GGGGCTAGTAAAATTGTAGTGTCT**TCAAGATCA**GAGAGAAGAATAGAAATGG 3'
SEQ ID N°8 (MUT225223-2)
   5' CCATTTCTATTCTTCTCTC**TGATCTTGA**AGACACTACAATTTTACTAGCCCC 3'

Des cellules d'*Escherichia coli* (XL-10 Gold® ultracompétentes, Stratagène, La Jolla, CA) ont été transformées successivement par les plasmides construits. Les transformants ont été cultivés à 37°C dans 5 ml de milieu LB supplémenté avec 50 µg/ml d'ampicilline et l'ADN plasmidique de ces clones a été extrait par le kit Genelute Plasmid Miniprep® (Sigma, USA) et analysé par séquençage.

### - Transformation

La souche de laboratoire FY834 *bdh1*Δ (*MAT*α *his3*Δ*200 ura3-52 leu2*Δ*1 lys2*Δ *trp1*Δ) a été transformée par pYES2-*BDH1*, pYES2-MUT 221, pYES2-MUT 222, pYES2-MUT 223. La méthode de transformation utilisée est celle de l'acétate de lithium décrite par Schiestl et Gietz (1989). Le milieu sélectif utilisé pour sélectionner les souches transformées par les plasmides est du YNB (0,67% Yeast nitrogen base, 2% glucose) supplémenté en histidine, leucine, lysine et tryptophane. L'absence d'uracile permet de conserver une pression de sélection pour les plasmides.

### - Conditions de culture

20 ml de milieu YNB (20 % galactose) ont été inoculés par 5 transformants, respectivement. La culture a été réalisée à 28°C pendant 2 jours.

### - Dosage d'activité enzymatique

L'activité butanediol déshydrogénasse a été déterminée à partir des extraits protéiques bruts à 25 °C en mesurant le changement d'absorbance à 340 nm. Une unité d'activité correspond à 1 µmol de cofacteur utilisé par min, basé sur un coefficient d'absorption de 6220 cm⁻¹ M⁻¹ à 340 nm pour NADH et NADPH. Les trajets optiques pour les réactions de réduction étaient 1 cm, 0,5 cm et 0,2 cm. Les dosages d'activité enzymatique ont été réalisés en présence de 50 mM d'acétoïne dans 33 mM NaH₂PO₄, pH 7,0/NaOH. Les paramètres cinétiques ont été obtenus par des dosages d'activité avec des concentrations en coenzyme de 1/3xKₘ à 10xKₘ de BDH pour NADH (0,055 mM).

L'activité BDH a été déterminée sur des extraits bruts de la souche exprimant la forme sauvage (pYES2*-BDH1)* et des souches exprimant les formes mutées. Les paramètres cinétiques des différentes formes ont également été déterminés (Tableau 2).
Les trois mutants présentent une affinité pour le NADH très fortement réduite, de 90 % en moyenne par rapport à celle de l'enzyme native.

**Tableau 2**

| NADH | AS (µmol/min.mg) | Kmₐ (mM) |
|---|---|---|
| BDH | 4,9 ± 0,8 | 0,04 ± 0,00 |
| BDH221 | 0,5 ± 0,1 | ND |
| BDH222 | 0,7 ± 0,2 | ND |
| BDH223 | 0,3 ± 0,2 | ND |
| | | |

| NADPH | AS (µmol/min.mg) | Kₘₐ (mM) |
|---|---|---|
| BDH | Non détecté | - |
| BDH221 | 1,0 ± 0,1 | 0,12 ± 0,02 |
| BDH222 | 4,6 ± 0,1 | 0,24 ± 0,14 |
| BDH223 | 5,6 ± 0,7 | 0,15 ± 0,01 |

Une réversion importante de la spécificité de cofacteur de NADH à NADPH est observée pour tous les mutants (Tableau 2). En effet, les mutants présentent tous une activité NADPH. dépendante. La constante d'affinité apparente (Kmₐ) pour le NADPH est du même ordre pour les trois mutants.

L'ensemble des données montre que les mutations réalisées diminuent très fortement l'activité NADH-dépendante de l'enzyme et introduisent une affinité pour le NADPH. Les trois mutants présentent une affinité proche pour le NADPH. En revanche, les formes double et triple mutantes sont particulièrement intéressantes en raison de leur meilleure activité spécifique comparativement au simple mutant.

Afin d'évaluer l'impact du changement de cofacteur de la BDH dans la souche modèle oenologique *V5ald6,* le gène *BDH1* portant la triple mutation, appelé *BDH1*₂₂₃*,* a été surexprimé dans cette souche par mutagénèse dirigée *in situ.* Deux oligonucléotides, dont l'un de 55 paires de bases (SEQ ID N°9) contenant les mutations cibles, ont été synthétisés et utilisés pour amplifier la région loxpKanMXloxp-*TDH3*p-*BDH1* d'une souche V5*ald6 BDH1.* Le fragment amplifié, contenant le marqueur KanMX et, sous contrôle du promoteur *TDH3,* le gène *BDH1* jusqu'à la région contenant les 3 mutations, a été utilisé pour transformer une souche *V5ald6.*

Cette stratégie a permis en une étape d'intégrer par recombinaison homologue les mutations cibles dans la séquence génomique de Bdh1p tout en surexprimant le gène muté.
SEQ ID N°1
SEQ ID N° 9
   5'GGCCATTTCTATTCTTCTCTCTGATCTTGAAGACACTACAATTTTACTGGCCCCC
Conditions expérimentales utilisées pour la PCR :

| | |
|---|---|
| Oligonucléotide (1) | 5µl (20 pmoles) |
| Oligonucléotide (2) | 5µl (20 pmoles) |
| Taq Buffer 10X + 15 mM MgCl₂ | 5µl |
| dNTP 2,5 mM | 4µl |
| ADN total *V5ald6 BDH1* | 2µl (50 ng/µl) |
| H₂O | 28,25µl |
| Taq | 0,75µl |

30 cycles (10 cycles : 20 sec 94°C, 20 sec 60°C, 3 min 72°C ; 20 cycles : 15 sec 94°C, 30 sec 60°C, 2 min + 20s/cycle 72°C).

Le produit de PCR obtenu a été précipité à l'éthanol en présence de sels. 4µg de l'ADN précipité ont été utilisés pour transformer les souches de levure *S. cerevisiae V5ald6*.

L'intégration a été vérifiée par digestion enzymatique du produit de PCR pendant 2 heures à 37°C par l'enzyme Bbs I en utilisant le mélange réactionnel suivant: 13,5 µl produit de PCR; 1,5 µl tampon NEB2 10X (Biolabs, USA) ; 0,25 µl (1,25 U) de Bbs I (Biolabs, USA).

L'activité enzymatique a été ensuite été déterminée sur l'extrait brut protéique d'une souche ayant intégré les mutations souhaitées. Pour chaque dosage, 20 µl d'extrait brut ont été utilisés en présence de 0,2 mM de NADH/NADPH et 50 mM d'acétoïne.

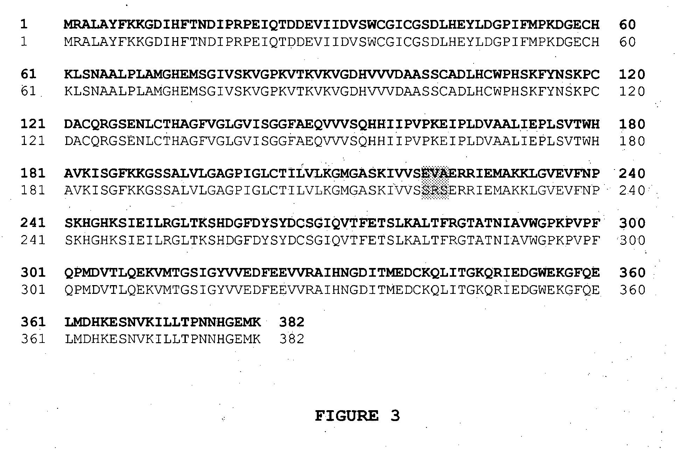

En parallèle, la région modifiée a été séquencée, ce qui a permis de vérifier que la séquence de BDH1 mutée est identique à celle du gène natif, à l'exception des mutations introduites, qui conduisent au changement de 3 acides aminés dans la protéine (Fig.3).

### Impact physiologique du changement de spécificité du cofacteur de Bdh1

La souche *V5ald6 BDH1* p*GPD1*, surexprimant la Bdh1 sauvage, a été comparée avec le clone ayant la Bdh1 NADPH-dépendante (V*5ald6 BDH1*₂₂₃ *pGPD1*) en fermentation oenologique sur moût synthétique à 200 g/l de glucose. Ces 2 souches ont été étudiées en fermentation en absence d'uracile (pression de sélection).

Dans ces conditions les profils de vitesse de fermentation et de biomasse sont identiques. Aucun effet significatif n'est observé sur les sous-produits fermentaires sauf une augmentation de la formation en α-cétoglutarate (de 480 mg/L à 620 mg/L), en OH-glutarate (de 1400 mg/L à 1620 mg/L) et en glycérol (de 30,2 g/L à 32,2 g/L) dans la souche surexprimant la Bdh mutée par rapport à la Bdh native. L'augmentation de la formation en glycérol peut s'expliquer par une plus grande disponibilité en NADH, du fait de l'utilisation préférentielle de NADPH par Bdh1₂₂₃. L'accumulation d'alpha-cétoglutarate peut être liée à la limitation de sa réduction en glutamate, réaction catalysée par la glutamate déshydrogénase Gdhlp NADPH dépendante. En revanche, la production d'OH-glutarate, un autre produit réduit de l'alpha-cétoglutaraté, pourrait résulter de la plus grande disponibilité en NADH. Ces observations montrent clairement un effet *in vivo* du changement de cofacteur de *BDH1* de NADH en NADPH.

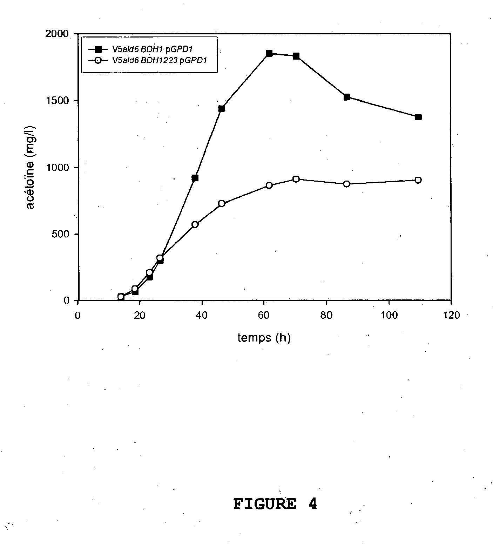

L'effet le plus marquant obtenu par le changement de spécificité de cofacteur est une réduction très significative de la production d'acétoïne chez la souche V5*ald6 BDH1₂₂₃ pGPD1* par rapport à la quantité produite par V5*ald6 BDH1* p*GPD1*. Dans les conditions testées, cette réduction est d'environ 400 mg/l (Fig. 4).

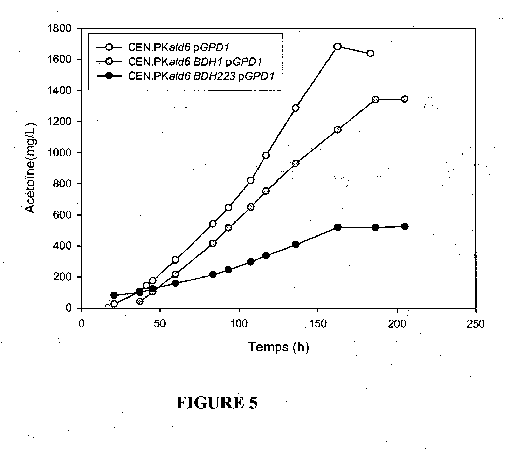

Les inventeurs ont d'autre part montré que cet effet était également obtenu dans un autre fond génétique, la souche *S. cerevisiae* de laboratoire CEN.PK. En effet, la surexpression de BDH₂₂₃ chez CEN.PK *ald6* p*GPD1*, qui produit 1638 mg/L d'acétoine sur moût synthétique contenant 50 g/L de glucose, conduit à une réduction de 68 % de la production d'acétoïne, alors que la surexpression de l'enzyme native réduit de seulement 18 % cette production. La quantité en 2,3-butanediol augmente de façon stoéchiométrique avec la diminution d'acétoïne (Figure 5).

Ceci montre clairement un effet physiologique lié au changement de spécificité de cofacteur de Bdh1 à des fortes concentrations en glycérol. Non seulement la quantité d'enzyme Bdh1 mais également la disponibilité en NADH est limitante dans des souches surproductrices de glycérol.

L'invention fournit ainsi des souches de levures sauvages et mutées, surproduisant la 2,3-butanediol déshydrogénase y compris des souches surproductrices de glycérol avec production d'acétate maîtrisée, accumulant du 2,3-butanediol en quantité importante et avec une production considérablement réduite d'acétoïne.

Comme le montrent les exemples ci-dessus, la surexpression de *BDH1 via* un remplacement *in situ* de son promoteur par celui de *TDH3* s'avère très efficace, puisqu'une augmentation d'activité d'un facteur 30 a été obtenue. L'analyse de ces souches au cours de la fermentation oenologique montre par ailleurs que le niveau d'expression de *BDH1* et d'activité enzymatique de BDH est bien un facteur limitant dans la conversion d'acétoïne en 2,3-butanediol dans des souches surproduisant du glycérol.

Ces résultats montrent que la conversion d'acétoïne en 2,3-butanediol est également limitée par la disponibilité en NADH. En effet, la surexpression d'une forme mutée de la BDH NADPH-dépendante engendre une diminution supplémentaire en acétoïne par rapport à une souche surexprimant l'enzyme sauvage.

Ainsi, la stratégie suivie selon l'invention dé surexpression de *BDH1* et de changement de spécificité de cofacteur de la BDH de NADH en NADPH permet une conversion extrêmement efficace de l'acétoïne en 2,3-butanediol.

La stratégie de surexpression de *BDH1*₂₂₃ peut être envisagée pour réduire l'acétoïne dans toute souche de levure modifiée ou non modifiée qui accumule ce composé, comme les souches surproductrices de glycérol. Un autre exemple est une souche surexprimant une NADH oxydase bactérienne. Il a en effet été montré que la surexpression du gène *NOXE* codant pour la NADH oxydase de *Lactococcus lactis* dans la souche V5 diminue la teneur intracellulaire en NADH, ce qui entraîne une baisse du rendement d'éthanol du fait de la limitation de l'activité alcool déshydrogénase. Chez cette souche, les carbones non dirigés vers la formation d'éthanol s'accumulent au niveau du noeud acétaldéhyde, en particulier l'acétoïne (Heux et al, 2006). L'expression d'une forme mutée de la BDH utilisant du NADPH en tant que cofacteur, en favorisant la conversion de l'acétoïne en 2,3-butanediol, pourrait diminuer la production de ce composé.

Les autres mutants décrits plus haut, qui présentent une affinité augmentée vis à vis du NADPH, en particulier le double mutant qui a les mêmes caractéristiques que le triple mutant, peuvent également s'avérer intéressants pour réduire l'acétoïne dans des conditions physiologiques.

L'expression d'une Bdh1p NADPH dépendante dans la levure permet de modifier l'équilibre des cofacteurs NADP/NADPH et, à ce titre, peut constituer un outil intéressant dans l'étude de l'équilibre d'oxydoréduction intracellulaire.

### BDH1 chez la levure : homologies de séquence inter- et intra-espèce.

Le changement de spécificité de la BDH a été conduit dans les souches S288C et V5 alors que le site de liaison du cofacteur diffère d'un acide aminé entre ces 2 souches. La souche V5 a en effet une valine à la position 222 alors que S288C a une isoleucine à la même position. Dans chacune de ses souches, l'introduction des mutations E221S, E221S/I(V)222R et E221S/I222R/A223S s'est avérée efficace et a permis d'obtenir un changement complet de spécificité de cofacteur de NADH en NADPH.

La conservation de ce site a été analysée dans d'autres souches de *Saccharomyces cerevisiae* dont le génome a été séquencé http://www.sanger.ac.uk/gbrowse/gbrowse/Saccharomyces/).
L'étude montre que les 12 souches analysées se divisent en 2 groupes, l'un (DBVPG1373, DBVPG1853, DBVPG6765, L_1374, L_1528 et SK1) caractérisé par la séquence 221 EVA 223 (séquence type V5), l'autre (DBVPG6044, S288C, Y55, YGPM, YPS128 et YPS606) par la séquence 221 EIA 223 (séquence type S288C). Ces observations indiquent que les effets de la mutagénèse dirigée décrits précédemment peuvent s'étendre aux autres souches de *S. cerevisiae*.

De la même manière, les homologies de séquence de Bdh1p (S288C) avec d'autres espèces de levure (http://cbi.labri.fr/Genolevures/path/) ont été recherchées. 5 gènes orthologues de *BDH1* ont été trouvés chez *Candida glabrata, Kluyveromyces lactis* et *Debaryomyces hansenii.* L'alignement des séquences protéiques montre une très forte conservation du site de liaison au NADH, les acides aminés 221 et 223 étant identiques à ceux de *S. cerevisiae*. La position de l'acide aminé 222 est plus variable, comme observé entre souches *S. cerevisiae*. Dans le cas des levures non *Saccharomyces,* cet acide aminé est une proline, de même famille (acides aminés aliphatiques hydrophobes) que l'isoleucine ou la valine.

Du fait de cette similarité de séquence, les changements d'acides aminés par mutagenèse dirigée décrits précédemment et leurs effets peuvent tout à fait s'appliquer à d'autres espèces levuriennes que *S. cerevisiae*.

### • Autre exemple de caractérisation des propriétés enzymatiques des formes mutées de Bdh1p.

- ***Transformation***
   La souche de laboratoire WV36-405 (Mata, ade2, ura3-52, trp1, adh1 Δ, adh2 Δ, adh3 Δ, adh4: : TRP1) (Atrian et al., 1990) a été également transformée par pYes2, pYes2-BDH1, pYes2-MUT 221, pYes2-MUT 222, pYES2-MUT 223 par la méthode de transformation à l'acétate de lithium décrite (Schiestl & Gietz, 1989). Le milieu sélectif utilisé pour sélectionner les souches transformées est du YNB (0.67% Yeast nitrogen base, 2% glucose) supplémenté en adénine.
- ***Conditions de culture et préparation des extraits bruts***
   20 ml de milieu YNB (2% galactose) ont été inoculés par les transformants. Les cellules ont été prélevées en phase de croissance, à DO (600nm) 2,5. Les cellules (10⁹) ont été suspendues dans 500 µl de tampon A (20 mM sodium phosphate (pH 7,0) contenant 1% de glycérol et 0,5 mM DTT) et broyées à l'aide de billes de verre. Après centrifugation à 12 000 tr / min pendant 5 min, le surnageant a été récupéré et utilisé pour les dosages enzymatiques.
- ***Dosage d'activité enzymatique***
   L'activité 2,3-butanediol déshydrogénase a été déterminée à partir des extraits bruts en mesurant le changement d'absorbance à 340nm comme décrit ci-dessous. Les dosages ont été réalisés en présence de 33 mM de phosphate de sodium (pH 7,0), 0,5 mg/L de BSA (sérum albumine bovine), d'acétoine et de 1 mM NAD (P) H. La concentration en protéines dans les extraits a été déterminée avec la méthode de Bradford (Bio-Rad). Les *Kₘ* et *Vₘ* pour le NAD(P)H (table 3) ont été déterminés en présence de 50 mM et 450 mM d'acétoïne pour Bdh1 and les mutants Bdh1 respectivement.

**Tableau 3.**

| | NADH | | |
|---|---|---|---|
| Enzyme | *K*ₘ (µM) | *Vₘ* (U/mg) | *Vₘ*/*Kₘ* (ml mg⁻¹ min⁻¹) |
| Bdh1 | 45 ± 4 | 94 ± 1,7 | 2090 ± 194,0 |
| Bdh1₂₂₁ | 700 ± 20 | 9 ± 0,1 | 12 ± 0,3 |
| Bdh1₂₂₂ | n.s | n.s | n.d |
| Bdh1₂₂₃ | n.s | n.s | n.d |
| | | | |

| | NADPH | | |
|---|---|---|---|
| Enzyme | *Kₘ* (µM) | *Vₘ*(U/mg) | *Vₘ*/*Kₘ* (ml mg⁻¹ min⁻¹) |
| Bdh1 | n.d | | |
| Bdh1₂₂₁ | 87 ± 6 | 15 ± 0,2 | 178 ± 12 |
| Bdh1₂₂₂ | 44 ± 4 | 59 ± 1,0 | 1347± 130 |
| Bdh1₂₂₃ | 44 ± 10 | 105 ± 1,5 | 2392 ± 157 |

| | | | |
|---|---|---|---|
| n.s : aucune saturation avec jusqu'à 1 mM NADH. n.d : non déterminé | | | |

**Les** activités spécifiques avec NADH et NADPH sont données dans le tableau 4.

**Tableau 4.**

| | Activité spécifique (U/mg) | |
|---|---|---|
| Enzyme | NADH (1mM) | NADPH (1mM) |
| Bdh1 | 92.0 ± 0.2 | 0.1 ± 0.0 |
| Bdh1₂₂₁ | 4:1 ± 1.4 | 13.7 ± 0.5 |
| Bdh1_{221,222} | 4.8 ± 1.7 | 54.7 ± 1.9 |
| Bdh1_{221,222,223} | 6.1 ± 0.1 | 93.2 ± 3.5 |

Les mutants présentent tous une activité NADPH dépendante (Tableaux 3 et 4). L'affinité pour le NADPH des formes double et triple mutantes (44 µM) est identique à celle de Bdh1 pour le NADH (45 µM). La comparaison des activités spécifiques de chaque enzyme en présence de 1 mM NAD (P) H (tableau 3) montre que la forme triple mutante a une activité sensiblement plus élevée pour le NADPH que le double mutant.

### Fonctionnement des formes mutantes in vivo

Afin d'évaluer le fonctionnement *in vivo* des formes Bdh1 mutantes NADPH dépendantes, la souche de laboratoire ENY*pgi1* (*MAT***a**, *ura3-52, leu2-3,* t*rp1*-289, *his-Δ*1, *MAL2-8c*, *MAL3, SUC3, pgi1*: :*KanMX*) (Heux et al, 2008) a été transformée par pYes2, pYes2-BDH1, pYes2-MUT 221, pYes2-MUT 222, pYES2-MUT 223. Les transformants ont été sélectionnés sur milieu minimal sans uracile contenant 2% de fructose et de 0,05% de glucose.

Des tests en goutte ont été réalisés à partir de cellules prélevées en phase exponentielle de croissance à DO₆₀₀ 1. Les cellules ont été diluées de 10 en 10, et 10µl de chaque dilution ont été déposés sur milieux gélosés YEP (Yeast extract 1%, Peptone 2%) 2% fructose plus 0,05% galactose ; YEP 0,5% galactose ou YEP 0,5% galactose plus 0,05 , 0,1 ou 0,2% acétoïne.

La souche ENY*pgi1* est délétée pour *PGI1* codant la phosphoglucose isomérase. Cette enzyme est située à la jonction de la glycolyse et de la voie des pentose phosphate (VPP). Dans ce mutant, le glucose 6-phosphate est entièrement redirigé vers la VPP, ce qui génère un excès de NADPH qui empêche la souche de se développer. Par contre, ce mutant peut se développer si on lui fournit un système de réoxydation du NADPH, par exemple fourni par expression de la transhydrogénase udha de *E. coli.*

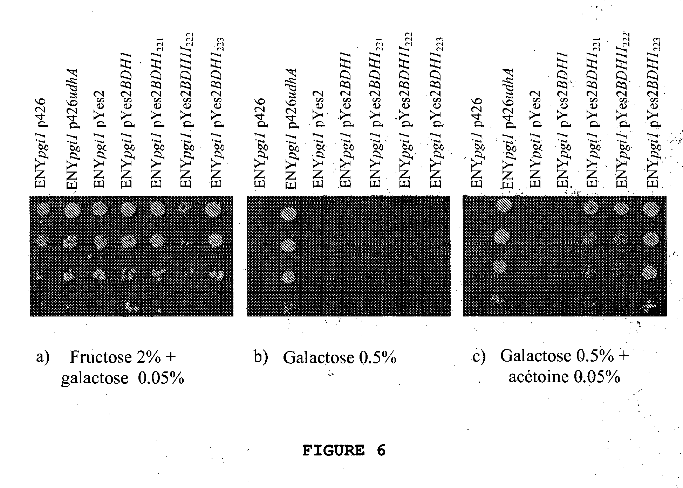

En effet, la figure 6 montre que ENYpgi ne peut croître sur galactose comme seule source de carbone, alors que l'expression de *udha* permet de restaurer sa croissance. Si on ajoute de l'acétoïne au milieu de culture (figure 6c), la croissance de ENYpgi1 exprimant les formes mutées NADPH dépendantes de Bdh1 est également restaurée. Il ressort de ces résultats que les formes BDH NADPH dépendantes sont capables de réoxyder le NADPH produit en excès par cette souche en NADP, ce qui démontre leur fonctionnalité *in vivo.*

### • surexpression de BDH1 pour réduire la formation de diacétyle

Le diacétyle (2,3-butanedione) est indésirable dans les boissons fermentées. Dans le vin, son seuil de détection varie entre 0,2 et 2,8 mg / L, et il est considéré comme indésirable au delà de 5 mg/L. Dans la bière, le diacétyle pose un problème majeur en raison de son seuil de détection très bas (0,1 ppm) et une longue étape de maturation est nécessaire pour éliminer ce composé.

Le diacétyle provient de la décarboxylation oxydative de l'α-acétolactate, un intermédiaire de la biosynthèse de l'isoleucine, leucine, valine, et peut être réduit en acétoïne. Il a été montré que Bdh1 a, *in vitro,* une activité de réduction du diacétyle. Afin d'étudier l'impact de Bdh1 sur le diacétyle *in vivo.*, un dosage de la concentration finale en diacétyle produit par les souches V5ald6pGPD1 et V5ald6BDH1pGPD1 dans l'expérience décrite figure 2a a été effectué.

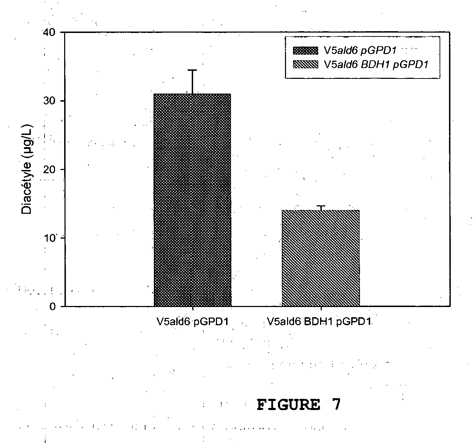

La concentration en diacétyle a été déterminée par SPME (solid-phase microextraction) en utilisant du diacétyle-d6 deutéré comme standard interne, et GC-MS (Hayasaka & Bartowsky, 1999). La surexpression de *BDH1* permet de réduire la production de diacétyle par un facteur 2 (figure 7). Des résultats similaires ont été obtenus avec une souche surexprimant la forme triple mutée de Bdhl.

### Références bibliographiques

Gonzalez, E, Fernandez, M R, Larroy, C, Sola, L, Pericas, M A, Pares, X, and Biosca, J A. 2000. Characterisation of a (2R,3R)-2,3-Butanediol Dehydrogenase as the Saccharomyces cerevisiae YAL060W Gene Product. J. Biol. Chem. 275: 35876-35885.
Remize, F, Roustan, J L, Sablayrolles, J M, Barre, P, and Dequin, S. 1999. Glycerol Overproduction by Engineered Saccharomyces cerevisiae Wine Yeast Strains Leads to Substantial Changes in By-Product Formation and to a Stimulation of Fermentation Rate in Stationary Phase. Appl. Environ. Microbiol. 65: 143-149.
Güldener, U, Heck, S, Fiedler, T, Beinhauer, J and Hegemann J.H. 1996. A new efficient gene disruption cassette for repeated use in budding yeast. Nucl. Ac. Res. 24: 2519-2524 Schiestl, R. H. and R.D. Gietz. 1989. High efficiency transformation of intact cells using single stranded nucleic acid as carrier. Curr. Genet. 16:339-446.
Bely, M., Sablayrolles J.M. and P. Barre. 1990. Automatic détection of assimilable nitrogen deficiencies during alcoholic fermentation in enological conditions. J. Ferm. Bioeng. 70:246-252.
Michnick S, Roustan JL, Remize F, Barre P, Dequin S, 1997. Modulationof glycerol and ethanolyields during alcoholic fermentation in Saccharomyces cerevisiae strains overexpressed or disrupted for GPD1 encoding glycerol-3-phosphate dehydrogenase. Yeast 13: 783-793.
Heux, S, Cachon, R, Dequin, S. 2005. Cofactor engineering in Saccharomyces cerevisiae: expression of a H2O-forming NADH oxidase and impact on metabolic flux redistribution. Metab. Eng.
Heux S., Sablayrolles JM, Cachon R. and Dequin S (2006) Engineering S. cerevisiae wine yeast with reduced ethanol production during fermentation under controlled microoxygenation conditions. Appl. Environ. Microbiol. 72:5822-5828
Lundquist, F. (1974). Acetaldehyd : Bestimmung Mit Aldehyd-dehydrogenase., Methods of Enzymatic Analysis., Academic Press, Inc., pp. 1509-1513.
Schiestl, R. H. & Gietz, R. D. (1989). High efficiency transformation of intact yeast cells using single stranded nucleic acids as a carrier. Curr Genet 16, 339-346.
Heux, S., Cadiere, A. & Dequin, S. (2008). Glucose utilization of strains lacking PGI1 and expressing a transhydrogenase suggests differences in the pentose phosphate capacity among Saccharomyces cerevisiae strains. FEMS Yeast Res. 8, 217-24.Hayasaka et Bartowsky (1999). Analysis of diacetyl in wine using solid-phase microextraction combined with gas chromatography-mass spectrometry. J Agric Food Chem 47, 612-617.

## Revendications

1. Souches de levures dans lesquelles le gène BDH1 codant pour la protéine Bdh1p ayant une activité butanediol déshydrogénase, est surexprimé par rapport à la souche initiale, **caractérisées en ce que** : - lesdites levures comprennent une ou plusieurs mutations dans le gène BDH1 afin de présenter une spécificité de cofacteur pour le NADPH au lieu du NADH, et catalysent la réduction de l'acétoïne en 2,3- butanediol selon un taux au moins 2 fois supérieur à celui de la souche initiale ; et
- **en ce que** la protéine Bdh1p codée par le gène BDH1 comprend une mutation en position 221, 222 ou 223, ou en positions 221 et 222, ou 221 et 223, ou 222 et 223, ou 221, 222 et 223 par référence à la souche de *Saccharomyces cerevisiae S288C*, la mutation en position 221 étant S, en position 222, R, et en position 223,S.

2. Souches de levures selon la revendication 1, **caractérisées en ce qu'**il s'agit de souches génétiquement transformées pour surexprimer BDH1 à l'aide d'un promoteur fort de levures.

3. Souches de levures selon l'une des revendications 1 à 2, **caractérisées en ce que** le gène BDH1 comporte une ou plusieurs mutations de manière à coder pour une protéine Bdh1p dans laquelle un ou plusieurs acides aminés sont mutés.

4. Souches de levures selon l'une des revendications 1 à 3, **caractérisées en ce qu'**elles comportent un gène GPD1 ou un gène GPD2 surexprimé.

5. Souches de levures selon l'une des revendications 1 à 4, **caractérisées en ce que** le gène ALD6 a été délété et le cas échéant ses copies.

6. Souches de levures selon l'une quelconque des revendications 1 à 5, **caractérisées en ce qu'**elles appartiennent au genre *Saccharomyces*.

7. Souches de levures selon la revendication 6, **caractérisées en ce qu'**il s'agit de *Saccharomyces cerevisiae, Saccharomyces bayanus*, *Saccharomyces uvarum et Saccharomyces kudriavzevii*.

8. Souches de levures selon l'une quelconque des revendications 1 à 5, **caractérisées en ce qu'**elles appartiennent au genre non Saccharomyces.

9. Souches de levures selon l'une quelconque des revendications 6 à 8, **caractérisées en ce qu'**il s'agit d'hybrides.

10. Méthode de réduction du taux d'acétoïne dans une souche de levure **caractérisée en ce qu'**elle comprend l'utilisation d'une souche de levure selon l'une quelconque des revendications 1 à 9.

11. Procédé de fermentation de jus de fruit, **caractérisé en ce qu'**il comprend l'addition dans un milieu de fermentation de souches de levure telles que définies dans l'une quelconque des revendications 1 à 9.

12. Procédé selon la revendication 11, **caractérisé en ce que** le jus de fruit est du jus de raisin.

13. Utilisation des souches de levures selon l'une quelconque des revendications 1 à 9, pour produire du 2,3-butanediol.

14. Utilisation des souches selon l'une quelconque des revendications 1 à 9, pour réduire la production de diacétyle, en particulier en brasserie.

## Patentansprüche

1. Hefestämme, in welchen das für das Protein Bdh1p, das eine Butandiol-Dehydrogenase-Aktivität besitzt, codierende Gen BDH1 im Vergleich zum Ursprungsstamm überexprimiert und **dadurch gekennzeichnet ist, dass**: - besagte Hefestämme eine oder mehrere Mutationen auf dem Gen BDH1 umfassen, um eine Cofaktor-Spezifizität für NADPH anstatt NADH aufzuweisen, und die Reduktion von Acetoin zu 2,3-Butandiol in einer verglichen mit dem Ausgangsstamm mindestens 2 Mal höheren Rate katalysieren; und dadurch, dass
- das durch das Gen BDH1 codierte Protein Bdhlp eine Mutation an Position 221, 222 oder 223, oder an den Positionen 221 und 222, oder 221 und 223, oder 222 und 223, oder 221, 222 und 223 aufweist im Vergleich zum Stamm Saccharomyces cerevisiae S288C wobei die Mutation an Position 221 S, an 222 R und an 223 S ist.

2. Hefestämme gemäß Anspruch 1, die **dadurch gekennzeichnet sind, dass** es sich um Hefen handelt, die genetisch verändert wurden, um die Überexpression von BDH1 mithilfe eines starken Promoters zu bewirken.

3. Hefestämme gemäß eines der Ansprüche 1 bis 2, **dadurch gekennzeichnet, dass** das Gen BDHI eine oder mehrere Mutationen besitzt, um für ein Protein Bdh1p zu codieren, in welchem eine oder mehrere Aminosäuren mutiert sind.

4. Hefestämme gemäß eines der Ansprüche 1 bis 3, **dadurch gekennzeichnet, dass** sie ein überexprimiertes Gen GPD1 oder GPD2 besitzen.

5. Hefestämme gemäß eines der Ansprüche 1 bis 4, **dadurch gekennzeichnet, dass** das Gen ALD6 und gegebenenfalls dessen Kopien deletiert wurden.

6. Hefestämme gemäß irgendeines der Ansprüche 1 bis 5, **dadurch gekennzeichnet, dass** sie der Gattung *Saccharomyces* angehören.

7. Hefestämme gemäß Anspruch 6, **dadurch gekennzeichnet, dass** es sich um *Saccharomyces cerevisiae, Saccharomyces bayanus, Saccharomyces uvarum* und *Saccharomyces kudriavzevii* handelt.

8. Hefestämme gemäß irgendeines der Ansprüche 1 bis 5, **dadurch gekennzeichnet, dass** sie der Gattung der Nicht-Saccharomyces angehören.

9. Hefestämme gemäß irgendeines der Ansprüche 6 bis 8, **dadurch gekennzeichnet, dass** es sich um Hybride handelt.

10. Methode zur Reduzierung des Acetoingehalts in einem Hefestamm, **dadurch gekennzeichnet, dass** sie die Verwendung eines Hefestamms gemäß irgendeines der Ansprüche 1 bis 9 umfasst.

11. Verfahren zur Vergärung von Saft aus Früchten, **dadurch gekennzeichnet, dass** es den Zusatz von Hefestämmen, wie diese in irgendeinem der Ansprüche 1 bis 9 definiert sind, in ein Fermentationsmedium umfasst.

12. Verfahren gemäß Anspruch 11, **dadurch gekennzeichnet, dass** der Fruchtsaft Traubensaft ist.

13. Verwendung von Hefestämmen gemäß eines der Ansprüche 1 bis 9 zur Produktion von 2.3-Butandiol.

14. Verwendung der Stämme gemäß irgendeines der Ansprüche 1 bis 9 zur Reduzierung der Produktion von Diacetyl, insbesondere in der Brauerei.

## Claims

1. Yeasts strains in which the BDH1 gene encoding Bdh1p, having an butanediol hydrogenase activity, is overexpressed relative to the initial strain, **characterized in that** :
- said yeasts comprise one or more mutations in the gene BDH1 in order to exhibit a cofactor specificity for NADPH instead of NADH, and catalyse the reduction of acetoin to 2,3-butanediol at a rate that is at least twice that of the initial strain, and
- **in that** the protein Bdh1p encoded by the gene BDH1 comprises a mutation at position 221, 222 or 223, or at positions 221 and 222, or 221 and 223, or 222 and 223, or 221, 222 and 223 with reference to the *Saccharomyces cerevisiae* S288C strain, the mutation at position 221 being S, at position 222, R, and at position 223, S.

2. Yeasts strains according to claim 1, **characterized in that** it is a strain genetically transformed strains so as to overexpress BDH1, by means of a strong yeast promoter.

3. Yeasts strains according to one of claims 1 to 2, **characterized in that** the gene BDH1 comprises one or more mutations in such a way as to encode a Bdh1p protein in which one or more amino acids are mutated.

4. Yeasts strains according to one of claims 1 to 3, **characterized in that** they comprise an overexpressed GPD1 gene or an overexpressed GPD2 gene.

5. Yeasts strains according to one of claims I to 4, **characterized in that** the ALD6 gene has been deleted, and optionally copies thereof are also deleted.

6. Yeasts strains according to any one of claims 1 to 5, **characterized in that** they belong to the *Saccharomyces* genus.

7. Yeasts strains according to claim 6, **characterized in that** it is *Saccharomyces cerevisiae, Saccharomyces bayanus, Saccharomyces uvarum* and *Saccharomyces kudriavzevii*.

8. Yeasts strains according to any one of claims 1 to 5, **characterized in that** they belong to the non*-Saccharomyces* genus.

9. Yeasts strains according to any one of claims 6 to 8, **characterized in that** it is hybrid.

10. Method for reducing the amount of acetoin in a yeast strain, **characterized in that** it comprises the use of a yeast strain according to any one of claims 1 to 9.

11. Process for fermenting fruit juice, **characterized in that** it comprises the addition, to a fermentation medium, of yeast strain as defined in any one of claims 1 to 9.

12. Process according to claim 11, **characterized in that** the fruit juice is grape juice.

13. Use of strains yeasts according to any one of claims 1 to 9 for producing 2,3-butanediol.

14. Use of strains yeasts according to any one of claims 1 to 9 for reducing diacetyl production, in particular in brewery.
